(19) Europäisches Patentamt
European Patent Office
Office européen des brevets

(11) EP 0 596 586 B1

(12) **EUROPÄISCHE PATENTSCHRIFT**

(45) Veröffentlichungstag und Bekanntmachung des Hinweises auf die Patenterteilung:
**22.01.1997 Patentblatt 1997/04**

(51) Int Cl.$^6$: **C07D 257/02**

(21) Anmeldenummer: **93250304.8**

(22) Anmeldetag: **05.11.1993**

(54) **Verfahren zur Herstellung von Metallkomplexen der N-beta-Hydroxy-alkyl-tri-N-carboxyalkyl-1,4,7,10-tetraazacyclododecan- und N-beta-Hydroxyalkyl-tri-N-carboxyalkyl-1,4,8,11-tetraazacyclotetradecan-Derivate**

Process for the production of metal complexes of N-beta-hydroxalkyl-tri-N-carboxyalkyl-1,4,7,10-tetraazacyclododecane and derivatives thereof

Procédé pour la préparation des complexes métalliques de la N-beta-hydroxyalkyle-tri-N-carboxyalkyle-1,4,7,10-tétraazacyclododécane et ses dérivés

(84) Benannte Vertragsstaaten:
**AT BE CH DE DK ES FR GB GR IE IT LI LU MC NL PT SE**

(30) Priorität: **06.11.1992 DE 4237943**

(43) Veröffentlichungstag der Anmeldung:
**11.05.1994 Patentblatt 1994/19**

(73) Patentinhaber: **SCHERING AKTIENGESELLSCHAFT**
**13353 Berlin (DE)**

(72) Erfinder:
- **Platzek, Johannes, Dr.**
  **D-14195 Berlin (DE)**
- **Gries, Heinz, Dr.**
  **D-10717 Berlin (DE)**

(56) Entgegenhaltungen:
**EP-A- 0 255 471**
**EP-A- 0 292 689**
**EP-A- 0 299 795**
**EP-A- 0 434 345**
**EP-A- 0 434 346**
**EP-A- 0 448 191**
**EP-A- 0 485 045**
**EP-A- 0 512 661**
**WO-A-89/01476**
**US-A- 4 889 931**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 596 586 B1

Printed by Jouve, 75001 PARIS (FR)

## Beschreibung

Die Erfindung betrifft das in den Patentansprüchen gekennzeichnete Verfahren zur Herstellung von Metallkomplexen der N-β-Hydroxyalkyl-tri-N-carboxyalkyl-1,4,7,10-tetraazacyclododecan- und N-β-Hydroxyalkyl-tri-N-carboxyalkyl-1,4,8,11-tetraazacyclotetradecan-Derivate.

Wegen ihrer Bedeutung als bildgebende Diagnostika (DE OS 36 25 417), insbesondere NMR-Diagnostika, ist die Herstellung von Metallkomplexen der N-β-Hydroxyalkyl-tri-N-carboxyalkyl-1,4,7,10-tetraazacyclododecan- und N-β-Hydroxyalkyl-tri-N-carboxyalkyl-1,4,8,11-tetraazacyclotetradecan-Derivate auf verschiedenen Wegen versucht worden, ohne daß bisher ein befriedigender Syntheseweg, insbesondere zu ihrer Herstellung im Betriebsmaßstab, gefunden werden konnte.

In der deutschen Offenlegungsschrift DE 36 25 417 Al ist ein Verfahren zur Herstellung von Metallkomplexen von N-substituierten Tri-N-carboxyalkyl-1,4,7,10-tetraazacyclododecan-Derivaten beschrieben, in dem Tri-N-ethoxycarbonylmethyl-1,4,7,10-tetraazacyclododecan-Derivate, die am vierten Stickstoffatom einen Substituenten tragen, nach Abspaltung der noch vorhandenen Carboxyschutzgruppen in die Metallkomplexe überführt werden. Das für dieses Verfahren benötigte cyclische Ausgangsmaterial wird durch gezielte Ringsynthese erhalten. Hierbei wird von zwei Reaktanten ausgegangen, die nach literaturbekannten Methoden [z.B. Richman, Org. Synthesis 58, 86 (1978); Atkins, J. Amer. Chem. Soc. 96, 2268 (1974)] cyclisiert werden: einer der beiden Reaktanten enthält ein geschütztes Stickstoffatom und trägt am Kettenende zwei Fluchtgruppen (z.B. Brom-, Mesyloxy-, Tosyloxy-, Triflat-, oder Alkoxycarbonylgruppen), die von den endständigen Stickstoffatomen des zweiten Reaktanten, einer - anders als der erste Reaktant - geschützten Triazaverbindung, nukleophil verdrängt werden.
Die im Verfahren der DE 36 25 417 Al verwendete Schutzgruppenchemie führt immer zu zusätzlichen Reaktionsschritten, in denen die Schutzgruppen wieder entfernt werden müssen. Außerdem fallen bei der Abspaltung große Mengen an Salzen an, die anschließend entsorgt werden müssen. Daher ist eine Vermeidung von Schutzgruppen gerade für Verfahren, die im Betriebsmaßstab genutzt werden sollen, wünschenswert.

Tweedle und Mitarbeiter beschreiben in der Europäischen Patentanmeldung 292 689 Al sowie in der Veröffentlichung Inorg. Chem. **1991**, 30, 1265-1269, daß ausgehend von der unsubstituierten makrocyclischen Verbindung 1,4,7,10-Tetraazacyclododecan über eine tricyclische Zwischenstufe die N-Formylverbindung erhalten werden kann. Diese noch drei ungeschützte Stickstoffatome tragende Verbindung kann nun mit Halogenessigester-Derivaten trialkyliert, deformyliert und in den tetrasubstituierten Tetraazamakrocyclus überführt werden. Nach Abspaltung der Carboxyschutzgruppen wird der tetrasubstituierte Komplexbildner erhalten, der zum Komplex umgesetzt werden kann.
Der von Tweedle und Mitarbeitern beschriebene Syntheseweg für die Metallkomplexe von N-substituierten Tri-N-carboxyalkyl-1,4,7,10-tetraazacyclododecan-Derivaten hat nicht nur den Nachteil einer unbefriedigend hohen Stufenzahl sondern ist wegen hohen Aufwandes zur Reinigung der Zwischenstufen sowie hoher Kosten für große Mengen notwendiger Ionenaustauscher für die Durchführung im Betriebsmaßstab wenig geeignet. Des weiteren ist eine Umsetzung von Tri-N-carboxymethyl-1,4,7,10-tetraazacyclododecan (DO3A, Verbindung (2) in Inorg. Chem. Vol. 30, No.6, 1991, 1267) mit primären Epoxiden zwar möglich jedoch sind die Ausbeuten für die Umsetzung mit sekundären Epoxiden deutlich schlechter und für eine Verwertung im Betriebsmaßstab schlecht geeignet.

Es besteht daher weiterhin die Aufgabe, ein Verfahren zur Herstellung von Metallkomplexen der N-β-Hydroxyalkyl-tri-N-carboxyalkyl-1,4,7,10-tetraazacyclododecan- und N-N-β-Hydroxyalkyl-tri-N-carboxyalkyl-1,4,8,11-tetraazacyclotetradecan-Derivate, das die Auswahl der im Verfahren zur Umsetzung benötigten Elektrophile möglichst nicht einschränkt und das vor allem für die Umsetzung größerer Substanzmengen geeignet ist, zur Verfügung zu stellen.

Diese Aufgabe wird durch das vorliegende Verfahren gelöst.

Es wurde gefunden, daß die Herstellung von Metall-Komplexen der N-β-Hydroxyalkyl-tri-N-carboxyalkyl-1,4,7,10-tetraazacyclododecan- und N-β-Hydroxyalkyl-tri-N-carboxyalkyl-1,4,8,11-tetraazacyclotetradecan-Derivate der allgemeinen Formel I

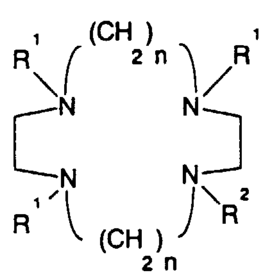

(I)

worin

$R^1$ $-CH_2-COOY$

Y Wasserstoff, ein Metallionenäquivalent eines Elementes der Ordnungszahlen 21-32, 37-39, 42-51 oder 57-83 mit der Maßgabe. daß mindestens zwei Substituenten Y für Metalläquivalente stehen

n die Ziffern 2 oder 3

$R^2$ eine

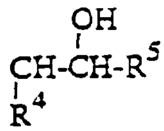

-Gruppe

$R^4$ und $R^5$ unabhängig voneinander jeweils für ein Wasserstoffatom, einen gegebenenfalls durch 1 bis 10 Sauerstoffatom(e), eine Phenylen-, Phenylenoxy- oder Phenylendioxygruppe unterbrochenen $C_1$-$C_{20}$-Alkylrest, der gegebenenfalls durch 1 bis 3 $C_1$-$C_6$-Alkyl-, 1-3 Trifluormethyl-, 1 bis 7 Hydroxy-, 1 bis 3 $C_1$-$C_7$-Alkoxy- oder - ($C_6$-$C_{10}$)-Ar($C_1$-$C_4$)alkoxy-, 1 bis 2 $CO_2R^6$-Reste, wobei

$R^6$ für ein Wasserstoffatom, eine $C_1$-$C_6$-Alkylgruppe, eine $C_6$-$C_{10}$-Aryl- oder eine $C_6$-$C_{10}$-Ar($C_1$-$C_4$)alkylgruppe steht,

stehen,

bedeuten, wobei die gegebenenfalls vorhandenen Hydroxyreste gegebenenfalls in geschützter Form vorliegen sowie deren Salze mit anorganischen und/oder organischen Basen, Aminosäuren oder Aminosäureamiden, enthaltend die Umsetzung von Intermediaten der allgemeinen Formel V

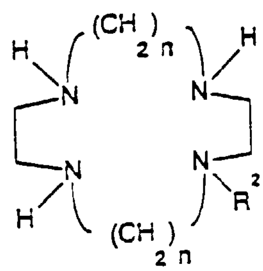

(V),

worin $R^2$ und n die oben genannten Bedeutungen haben, zu Verbindungen der allgemeinen Formel VII

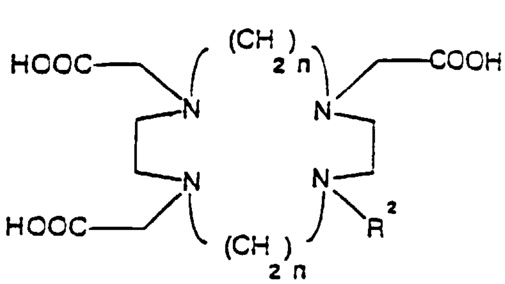

(VII),

worin $R^2$ und n die oben genannten Bedeutungen haben,

dadurch gekennzeichnet, daß die aus 1,4,7,10-Tetraazacyclododecan oder 1,4,8,11-Tetraazacyclotetradecan erhaltene Verbindung der allgemeinen Formel II

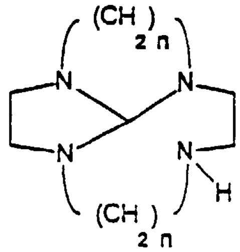

(II)

worin

n für die Ziffern 2 oder 3 steht
mit einem Epoxid der allgemeinen Formel III

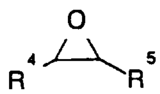

(III),

worin
$R^4$ und $R^5$ die oben angegebene Bedeutung haben, wobei gegebenenfalls vorhandene Hydroxy- oder Carboxygruppen gegebenenfalls geschützt sind, zu einem Intermediat der allgemeinen Formel IV

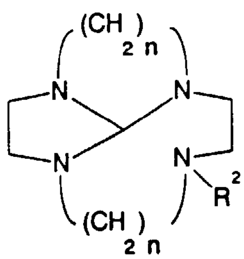

(IV),

worin
$R^2$ die oben angegebene Bedeutung hat,
wobei gegebenenfalls vorhandene Hydroxygruppen und/oder Carboxygruppen gegebenenfalls geschützt sind,
umgesetzt wird,
dieses zu einem Intermediat V verseift

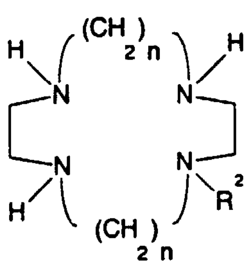

(V),

und dieses in Gegenwart einer Base, mit einer Verbindung der Formel VI

$$X\text{-}CH_2\text{-}COOZ \qquad (VI),$$

worin

X eine Abgangsgruppe,
Z Wasserstoff, eine Carboxyschutzgruppe oder ein Metallkation

bedeuten,
gegebenenfalls nach Schutz von Hydroxy- oder Carboxygruppen in einem polaren Lösungsmittel bei -10°C bis 170°C innerhalb von 1-100 Stunden umgesetzt wird, gegebenenfalls Schutzgruppen abgespalten werden und der so erhaltene Komplexbildner der Formel VII,

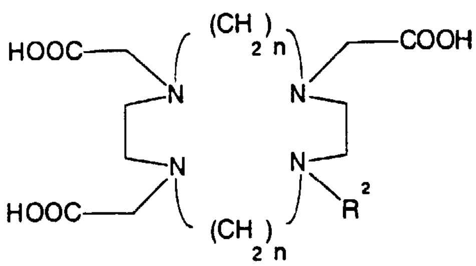

(VII)

mit einem Metalloxid oder Metallsalz eines Elementes der Ordnungszahlen 21-32, 37-39, 42-51 oder 57-83 umgesetzt wird und gegebenenfalls noch vorhandene Wasserstoffatome durch Kationen anorganischer und/oder organischer Basen, Aminosäuren oder Aminosäureamide substituiert werden oder die noch vorhandenen Säuregruppen ganz oder teilweise in Ester oder Amide überführt werden.

Das erfindungsgemäße Verfahren zeigt folgende überraschende Effekte:

1) Die gewünschten Metallkomplexe der allgemeinen Formel (I) werden in einer "Eintopfreaktion" unter günstigen betriebstechnischen Bedingungen in höherer Ausbeute als bei Verfahren des Standes der Technik erhalten.
2) Die Intermediate werden in so überraschend hoher Reinheit erhalten, daß auf ihre Isolierung und Reinigung verzichtet werden kann.
3) Im Gegensatz zu dem Verfahren nach Tweedle et al. verläuft dieUmsetzung sogar mit sekundären Epoxiden mit guten Ausbeuten.
4) Im Vergleich mit dem Stand der Technik beinhaltet das erfindungsgemäße Verfahren weniger Stufen.
5) Die Verwendung von Schutzgruppen ist für das erfindungsgemäße Verfahren zwar möglich aber nicht notwendig.

Bevorzugt wird ein Verfahren, das dadurch gekennzeichnet ist, daß eine Verbindung der Formel (I)

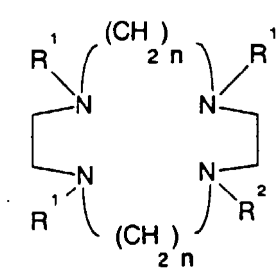

(I)

worin

$R^1$ $-CH_2-COOY$

Y ein Metallionenäquivalent eines Elementes des Ordnungszahlen 21-32, 37-39, 42-51 oder 57-83 mit der Maßgabe. daß mindestens zwei Substituenten Y für Metalläquivalente stehen

n die Ziffer 2

$R^2$ eine

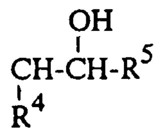

-Gruppe

$R^4$ und $R^5$ unabhängig voneinander jeweils für ein Wasserstoffatom, einen gegebenenfalls durch 1 bis 5 Sauerstoffatom(e), eine Phenylen-, Phenylenoxy- oder Phenylendioxygruppe unterbrochenen $C_1$-$C_{10}$-Alkylrest, der gegebenenfalls durch 1 bis 3 $C_1$-$C_6$-Alkyl-, 1-3-Trifluormethyl, 1 bis 5 Hydroxy-, 1 bis 3 $C_1$-$C_7$-Alkoxy- oder 1 bis 2 $CO_2R^6$-Reste, wobei

$R^6$ für ein Wasserstoffatom, eine $C_1$-$C_6$-Alkylgruppe, oder eine Benzylgruppe steht,

stehen,

bedeuten, erhalten wird.

Besonders bevorzugt wird ein Verfahren zur Herstellung von Metallkomplexen von 10-(1-Hydroxymethyl-2,3-dihydroxypropyl)-1,4,7-tris(carboxymethyl)-1,4,7,10-tetraazacyclododecan und von Metallkomplexen von 10-(2-Hydroxypropyl)-1,4,7-tris(carboxymethyl)-1,4,7,10-tetraazacyclododecan.

Ganz besonders bevorzugt wird ein Verfahren zur Herstellung von Gadolinium- oder Dysprosium-Komplexen von 10-(1-Hydroxymethyl-2,3-dihydroxypropyl)-1,4,7-tris(carboxymethyl)-1,4,7,10-tetraazacyclododecan und Gadolinium- oder Dysprosium-Komplexen von 10-(2-Hydroxypropyl)-1,4,7-tris(carboxymethyl)-1,4,7,10-tetraazacyclododecan.

Bevorzugte Reste $R^4$ und $R^5$ sind das Wasserstoffatom, die Methyl-, Ethyl-, 2-Hydroxyethyl-, 2-Hydroxy-1(hydroxymethyl)ethyl-, 1-(Hydroxymethyl)ethyl-, Propyl-, Isopropyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-, 2-Hydroxy-2-methylbutyl-, 3-Hydroxy-2-methylbutyl-, 4-Hydroxy-2-methylbutyl-, 2-Hydroxyisobutyl-, 3-Hydroxyisobutyl-, 2,3,4-Trihydroxybutyl-, 1,2,4-Trihydroxybutyl-, Pentyl-, Cyclopentyl-, 2-Methoxyethyl-, Hexyl-, Decyl-, Tetradecyl-, Triethylenglycolmethylether-, Tetraethylenglykolmethylether- und Methoxybenzylgruppe sowie die

$-CH_2-O-C_{11}H_{22}-OH-$,
$-CH_2-O-C_6H_4-O-(CH_2CH_2O)_2-CH_3-$
$-CH_2-O-C_6H_4-O-(CH_2CH_2O)_3-C_5H_{11}-$,
$-CH_2-O-C_6H_4-O-C_4H_8-OH-$,
$-(CH_2CH_2O)_5-CH_3-$,
$-C_9H_{18}-COOH-$,
$-C_9H_{18}-OH-$,
$-CH_2-O-C_6H_4-O-C_6H_{12}-COOH-$,
$-CH_2-O-C_6H_4-O-C_4H_8-O-CH_2-CHOH-CH_2OH-$,
$-(CH_2CH_2-O)_3-C_5H_{11}-$,
$-CH_2-O-C_{10}H_{20}-COOH-$,
$-CH_2-O-C_6H_4-Cl-$,
$-CH_2-O-C_6H_4-NO_2-$,
$-CH_2-O-C_6H_3Cl_2-$,
$-CH_2-O-C_6H_4-O-CH_2-COOH-$,
$-CH_2-O-C_6H_4-O-CH_2-COOH-$ und
$-CH_2-O-C_6H_4-C_5H_{11}$-Gruppe.

Als Alkoxysubstituenten in $R^4$ und $R^5$ sind geradkettige oder verzweigte Reste mit 1 bis 6 bzw. 1 bis 7 C-Atomen zu verstehen wie z.B. Methoxy, Ethoxy, Propoxy, Isopropoxy.

Als Alkylgruppe $R^6$ mit 1-6 Kohlenstoffatomen kommen geradkettige oder verzweigte Alkylgruppen in Betracht wie beispielsweise Methyl, Ethyl, Propyl, Isopropyl, Butyl, Isobutyl, tert.-Butyl. Besonders bevorzugt werden Methyl, Ethyl, tert.-Butyl.

Bevorzugte Arylgruppen und Aralkylgruppen $R^6$ sind die Phenyl-, Naphthyl- und die Benzylgruppe.

Besonders bevorzugte Reste $R^6$ sind Wasserstoff, der Methylrest oder der Benzylrest.

Die als Edukte verwendeten Tetraazatricyclotridecane- bzw. -pentadecane der allgemeinen Formel II sind nach literaturbekannten Methoden zugänglich, indem man 1,4,7,10-Tetraazacyclododecan bzw. 1,4,8,11-Tetraazacyclotetradecan mit Dimethylformamid-dimethylacetal umsetzt (US-Patentschriften 4,085,106 und 4,130,715). Vorteilhafterweise bezieht man diesen Reaktionsschritt in das erfindungsgemäße Verfahren ein.

<u>Reaktionsbedingungen für die Verfahrensschritte</u>

1) (II) + (III) → (IV) → (V)

Die Umsetzung der tricyclischen Zwischenstufe (II) mit einem Epoxid der Formel (III) erfolgt mit oder ohne Lösungsmittel zwischen 0°C und 220°C, vorzugsweise zwischen Raumtemperatur und 210°C, innerhalb von 1 bis 48 Stunden, vorzugsweise von 5 bis 12 Stunden, gegebenenfalls bei einem Druck bis zu 100 atm..

Das die Verbindung (IV) enthaltende Reaktionsgemisch wird nach Abkühlung auf -20°C bis 80°C, vorzugsweise 0°C bis 30°C, mit einem Gemisch Wasser/organisches Lösungsmittel versetzt und 0,5 bis 12 Stunden, vorzugsweise 0,5 bis 3 Stunden bei -20°C bis Raumtemperatur, vorzugsweise 0°C bis Raumtemperatur, gerührt.

Durch Zugabe einer anorganischen Base oder einer Säure wird bei 0°C bis 150°C, vorzugsweise Raumtemperatur bis 120°C, innerhalb von 1 bis 72, vorzugsweise 6 bis 24 Stunden, unter Rühren - gegebenenfalls gefolgt von anschließender Schutzgruppenentfernung in an sich üblicher Weise - zum Intermediat der Formel V umgesetzt. Dieses kann auch falls gewünscht als Salz, vorzugsweise als Hydrochlorid, isoliert werden.

Als Lösungsmittel sind für die Umsetzung von Verbindungen der Formel II mit Verbindungen der Formel III vor allem aprotische Lösungsmittel wie zum Beispiel Benzol, Toluol, Dichlormethan, Tetrahydrofuran, Dioxan, Acetonitril, Dimethylformamid, Dimethylacetamid, Dimethylsulfoxid, Hexan oder Diethylether geeignet.

Die im Gemisch mit Wasser eingesetzten Lösungsmittel können z.B. Methanol, Ethanol, Isopropanol, Tetrahydrofuran, Dioxan sein.

Als Base oder Säure sind beispielsweise geeignet Alkali- und Erdalkalihydroxide, Alkali- und Erdalkalicarbonate oder Mineralsäuren wie z.B. Salz- oder Schwefelsäure oder Methansulfonsäure.

2) (V) + (VI) → (VII)

Die bei der weiteren Umsetzung des Intermediates der Formel V mit einer Verbindung der Formel VI als Säurefänger zugesetzten Basen können tertiäre Amine (z.B. Triethylamin, Trimethylamin, N,N-Dimethylaminopyridin, 1,5-Diazabicyclo[4.3.0]-non-5-en(DBN), 1,5-Diazabicyclo[5.4.0]-undec-5-en(DBU), Alkali- oder Erdalkalicarbonate, Alkali- oder Erdalkalihydrogencarbonate, oder Alkali- oder Erdalkalihydroxide (z.B. Lithium-, Natrium-, Magnesium-, Calcium-, Barium-, Kalium-, -carbonat, -hydroxid und -hydrogencarbonat) sein.

Die Reaktion erfolgt in polaren Lösungsmitteln wie zum Beispiel Wasser, Acetonitril, Dimethylformamid, Dimethylsulfoxid, Hexamethylphosphorsäuretriamid oder Tetrahydrofuran sowie in Alkoholen mit einer Kettenlänge mit bis zu 8 C-Atomen wie z.B. Methanol, Ethanol, Propanol, Isopropanol, n-Butanol, Isobutanol, tert.-Butanol.

Die Umsetzung wird bei Temperaturen von -10°C - 170°C, bevorzugt bei 0° - 120°C, besonders bevorzugt bei 40° - 100°C innerhalb von 0,5-48 Stunden , vorzugsweise 3-24 h, durchgeführt.

Die gegebenenfalls durchgeführte Einführung oder Spaltung von Schutzgruppen der Carboxyl- oder Hydroxyfunktionen erfolgt nach literaturbekannten Methoden.

Als Säureschutzgruppen kommen niedere Alkyl-, Aryl- und Aralkylgruppen, beispielsweise die Methyl-, Ethyl-, Propyl-, n-Butyl-, tert.-Butyl-, Phenyl-, Benzyl-, Diphenylmethyl-, Triphenylmethyl-, bis(p-Nitrophenyl)-methylgruppe sowie Trialkylsilylgruppen in Frage.

Die Abspaltung der Schutzgruppen erfolgt nach den dem Fachmann bekannten Verfahren, beipielsweise durch Hydrolyse, Hydrogenolyse, alkalische Verseifung der Ester mit Alkali in wäßrig-alkoholischer Lösung bei Temperaturen von 0 bis 50°C, saure Verseifung mit Mineralsäuren oder im Fall von z.B. tert.-Butylestern mit Hilfe von Trifluoressigsäure. Als Metallkationen Z sind Metallkationen der Elemente der Alkali- bzw. Erdalkalimetalle möglich.

Als Hydroxyschutzgruppen kommen z.B. die Benzyl-, 4-Methoxybenzyl-, 4-Nitrobenzyl-, Trityl-, Diphenylmethyl-,

Trimethylsilyl-, Dimethyl-t-butylsilyl-, Diphenyl-t-butylsilylgruppen infrage.
Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Falle von Polyolen können die Hydroxygruppen auch in Form von Ketalen mit z.B. Aceton, Acetaldehyd, Cyclohexanon oder Benzaldehyd geschützt sein.
Die Hydroxyschutzgruppen können nach den dem Fachmann bekannten Literaturmethoden, z.B. durch Hydrogenolyse, reduktive Spaltung mit Lithium/Ammoniak, Säurebehandlung der Ether und Ketale oder Alkalibehandlung der Ester freigesetzt werden (siehe z.B. "Protective Groups in Organic Synthetics", T.W. Greene, John Wiley and Sons 1981).

Die intermediär erhaltenen Komplexbildner der Formel VII können in vorteilhafter Weise durch Ionenaustauscher aufgereinigt werden. Hierzu eignen sich im besonderen Kationenaustauscher (in der $H^+$-Form), die zuerst mit Wasser gewaschen werden und dann mit wässriger Ammoniak-Lösung eluiert das gewünschte Produkt liefern. Als besonders günstige Austauscher haben sich IR 120 ($H^+$) als auch AMB 252c ($H^+$) sowie Reillex® erwiesen. Verwendet man Reillex® wird der Komplexbildner mit Wasser oder wässrigen Alkoholen eluiert.

3) (VII) → (I)

Die Herstellung der Metallkomplexe gemäß Formel I erfolgt in an sich bekannter Weise (siehe z.B.: EP 292689 oder US 4,889,931), indem man die Komplexbildner der allgemeinen Formel VII mit einem Metalloxid oder Metallsalz eines Elementes der Ordnungszahlen 21-32, 37-39, 42-51 oder 57-83 vorzugsweise in Wasser und/oder in wässrigen Lösungen niederer Alkohole (wie z.B. Methanol, Ethanol oder Isopropanol) bei Temperaturen von 20°C-110°C, vorzugsweise 80°C-100°C umsetzt. Als besonders günstig hat sich die Zugabe von 0,1-4 Äquivalente vorzugsweise 0,5-2 Äquivalente einer anorganischen oder organischen Säure, vorzugsweise Essigsäure erwiesen.

Die so erhaltenen Metallkomplex-Lösungen können vorteilhafterweise durch Behandlung an einer Ionenaustauscher-Kaskade oder im Batch, bestehend aus saurem Kationenaustauscher ($H^+$-Form) und basischem Anionenaustauscher ($OH^-$-Form), bevorzugt IR 120 $H^+$, AMB 252c /IRA 67 aufgereinigt werden.

Die Endreinigung wird vorteilhafterweise durch eine Kristallisation aus einem niederen Alkohol, oder einem Alkohol-Wasser-Gemisch vorgenommen. Als Alkohole seien Methanol, Isopropanol, bevorzugt hingegen Ethanol genannt.

Die eingesetzten Metallsalze können beispielsweise Nitrate, Acetate, Carbonate, Chloride oder Sulfate sein. Das in dem verwendeten Metalloxid oder Metallsalz enthaltene Metall kann ein Element der Ordnungszahlen 21-32, 37-39, 42-51 oder 57-83 sein.

Die nachfolgenden Beispiele dienen zur näheren Erläuterung des Erfindungsgegenstands.

**Beispiel 1**

Gadolinium-Komplex von 10-(1-Hydroxymethyl-2,3-dihydroxypropyl)-1,4,7-tris(carboxymethyl)-1,4,7,10-tetraazacyclododecan

a) Zu 20 kg (116,10 mol) Cyclen (= 1,4,7,10 Tetraazacyclododecan) in 140 l Toluol gibt man (unter Stickstoff) 20 l (150,55 mol) Dimethylformamiddimethylacetal. Es wird langsam hochgeheizt und das Azeotrop aus Methanol/Dimethylamin/Toluol abdestilliert. Anschließend wird durch Anlegen eines Vakuums das Lösungsmittel vollständig abdestilliert. Das zurückbleibende Öl läßt man auf 50°C abkühlen und tropft dann 18,7 kg (- 95%ig) (123,22 mol) 4,4-Dimethyl-3,5,8-trioxabicyclo-(5.1.0)-octan zu (unter Stickstoff). Dann wird 24 Stunden bei 120°C gerührt. Man kühlt auf Raumtemperatur ab und tropft ein Gemisch aus 100 l Wasser/150 l Methanol zu. Dann wird 1 Stunde bei 50°C gerührt und 13,93 kg (348,3 mol) Natriumhydroxid zugegeben. Anschließend wird 8 Stunden unter Rückfluß erhitzt. Die Lösung wird weitgehend zur Trockne eingedampft, 200 l Wasser zugegeben und nochmals ca. 100 l Wasser abdestilliert. Es werden nochmals 100 l Wasser zugegeben und diese Lösung einmal mit 200 l n-Butanol und anschließend mit 50 l n-Butanol extrahiert. Die vereinigten Butanolphasen werden im Vakuum zur Trockne eingeengt und der Rückstand in 300 l Wasser aufgenommen. Dann wird 2 mal mit je 50 l Essigester extrahiert. Die Wasserphase wird abgetrennt und auf ca. 200 l Volumen eingeengt.

b) 43,84 kg (464,4 mol) Chloressigsäure werden in 150 l Wasser gelöst und mit 50 %iger aqu. Natronlauge auf pH 7 gestellt. Zu dieser Lösung gibt man die auf ca. 200 l Volumen eingeengte Wasserphase und erwärmt auf 80°C. Der pH-Wert wird durch Zugabe von 50 %iger aqu. Natronlauge zwischen pH 9,5 - 10 gehalten. Nach 10 Stunden gibt man weitere 10,96 kg (116,1 mol) Chloressigsäure (zuvor wie oben beschrieben in 35 l Wasser mit 50 %iger aqu. Natronlauge neutralisiert) zu. Es wird 12 Stunden bei 80°C gerührt und der pH-Wert zwischen 9,5 und 10 gehalten. Man läßt auf Raumtemperatur abkühlen und stellt mit konz. Salzsäure auf pH 0,8. Anschließend

wird 2 Stunden bei 60°C gerührt. Die Lösung wird im Vakuum weitgehend zur Trockne eingeengt. Der Rückstand wird 2 mal mit einem Gemisch aus 200 l Methanol/200 l Ethanol versetzt und zur Trockne eingedampft. Anschließend wird der Rückstand 1 Stunde bei 50°C mit 400 l Methanol ausgerührt. Man filtriert vom ausgefallenen Natriumchlorid ab, wäscht 2 mal mit 100 l Methanol nach und engt die vereinigten Filtrate im Vakuum zur Trockne ein. Der Rückstand wird in 200 l Wasser gelöst und auf eine Ionenaustauschersäule, gefüllt mit AMB 252c, gegeben. Man wäscht mit reichlich Wasser und eluiert das Produkt mit einer 10 %igen aqu. Ammoniak-Lösung. Die produkthaltigen Fraktionen werden in Vakuum weitgehend zur Trockne eingedampft.

c) Der Rückstand wird in 200 l Wasser gelöst und 16,31 kg (45 mol) Gadoliniumoxid zugegeben. Man kocht 3 Stunden unter Rückfluß. Anschließend setzt man 2 l Eisessig zu und kocht weitere 2 Stunden unter Rückfluß. Es wird 5 kg Aktivkohle zugegeben und 1 h bei 90°C gerührt. Die Lösung wird filtriert und das Filtrat mehrmals über eine Ionenaustauscher-Säulen-Kaskade (bestehend aus IRA 67 ($OH^-$-Form)) AMB 252c ($H^+$-Form) gegeben (unter HPLC-Kontrolle). Das Eluat wird auf ein Volumen von 300 l eingeengt und mit je 2 l saurem Ionenaustauscher IR 120 ($H^+$) sowie basischem Austauscher IRA 67 ($OH^-$) 3 Stunden lang gerührt. Man filtriert vom Austauscher ab und wäscht 2 mal mit 10 l Wasser nach. Zum Filtrat setzt man 5 kg Aktivkohle zu und rührt 2 Stunden bei 80°C. Die Lösung wird filtriert und das Filtrat im Vakuum eingeengt. Der Rückstand wird aus 95 % aqu. Ethanol (ca. 400 l) umkristallisiert. Man saugt den Niederschlag ab, wäscht 2 mal mit 80 l reinem Ethanol nach und trocknet ihn 48 Stunden bei 70°C im Trockenschrank.
Ausbeute: 47,6 kg (65,0 % d. Th.) (auf Wasser korrigiert/bezogen auf Cyclen) farbloses kristallines Pulver

Wassergehalt: 4,1 %

| Elementaranalyse (bezogen auf wasserfreie Substanz): | | | | |
|---|---|---|---|---|
| ber. | C 35,75 | H 5,17 | N 9,27 | Gd 26,00 |
| gef. | C 35,92 | H 5,24 | N 9,20 | Gd 25,83 |

**Beispiel 2**

Dysprosium-Komplex von 10-(1-Hydroxymethyl-2,3-dihydroxypropyl)-1,4,7-tris(carboxymethyl)-1,4,7,10-tetraazacyclododecan

In analoger Weise kann der entsprechende Dysprosium-Komplex hergestellt werden, wenn anstelle von Gadoliniumoxid entsprechend 16,78 kg (45 mol) Dysprosiumoxid umgesetzt werden.
Ausbeute: 46,36 kg (62,7 % d. Th.) (auf Wasser korrigiert/bezogen auf Cyclen)

Wassergehalt: 3,9 %

| Elementaranalyse (bezogen auf wasserfreie Substanz): | | | | |
|---|---|---|---|---|
| ber. | C 35,44 | H 5,12 | N 9,19 | Dy 26,64 |
| gef. | C 35,35 | H 5,21 | N 9,11 | Dy 26,57 |

**Beispiel 3**

Gadoliniumkomplex von 10-(2-Hydroxypropyl)-1,4,7-tris(carboxymethyl)-1,4,7,10-tetraazacyclododecan

a) Zu 20 kg (116,10 mol) Cyclen (= 1,4,7,10 Tetraazacyclododecan) in 140 l Toluol gibt man (unter Stickstoff) 20 l (150,55 mol) Dimethylformamiddimethylacetal. Es wird langsam hochgeheizt und das Azeotrop aus Methanol/Dimethylamin/Toluol abdestilliert. Anschließend wird durch Anlegen eines Vakuums das Lösungsmittel vollständig abdestilliert. Da zurückbleibende Öl läßt man auf 50°C abkühlen und tropft dann 10,11 kg (174,15 mol) Propylenoxid zu (unter Stickstoff). Dann wird 24 Stunden unter Rückfluß gekocht und anschließend überschüssiges Propylenoxid in Vakuum abdestilliert. Man kühlt auf Raumtemperatur ab und tropft ein Gemisch aus 100 l Wasser/150 l Methanol zu. Dann wird 1 Stunde bei 50°C gerührt und 13,93 kg (348,3 mol) Natriumhydroxid zugegeben. Anschließend wird 8 Stunden unter Rückfluß erhitzt. Die Lösung wird weitgehend zur Trockne eingedampft, 200 l Wasser zugegeben und nochmals ca. 100 l Wasser abdestilliert. Es werden nochmals 100 l Wasser zugegeben

und diese Lösung einmal mit 200 l n-Butanol und anschließend mit 100 l n-Butanol extrahiert. Die vereinigten Butanolphasen werden im Vakuum zur Trockne eingeengt und der Rückstand in 300 l Wasser aufgenommen. Dann wird 2 mal mit je 50 l Essigester extrahiert. Die Wasserphase wird abgetrennt und auf ca. 200 l Volumen eingeengt.

b) 43,84 kg (464,4 mol) Chloressigsäure werden in 150 l Wasser gelöst und mit 50 %iger aqu. Natronlauge auf pH 7 gestellt. Zu dieser Lösung gibt man die auf ca. 200 l Volumen eingeengte Wasserphase und erwärmt auf 80°C. Der pH-Wert wird durch Zugabe von 50 %iger aqu. Natronlauge zwischen pH 9,5 - 10 gehalten. Nach 10 Stunden gibt man weitere 10,96 kg (116,1 mol) Chloressigsäure (zuvor wie oben beschrieben in 35 l Wasser mit 50 %iger aqu. Natronlauge neutralisiert) zu. Es wird 12 Stunden bei 80°C gerührt und der pH-Wert zwischen 9,5 und 10 gehalten. Man läßt auf Raumtemperatur abkühlen und stellt mit konz. Salzsäure auf pH 0,8. Die Lösung wird im Vakuum weitgehend zur Trockne eingeengt. Der Rückstand wird 2 mal mit einem Gemisch aus 200 l Methanol/200 l Ethanol versetzt und zur Trockne eingedampft. Anschließend wird der Rückstand 1 Stunde bei 50°C mit 400 l Methanol ausgerührt. Man filtriert vom ausgefallenen Natriumchlorid ab, wäscht 2 mal mit 100 l Methanol nach und engt die vereinigten Filtrate im Vakuum zur Trockne ein. Der Rückstand wird in 200 l Wasser gelöst und auf eine Ionenaustauschersäule, gefüllt mit AMB 252c, gegeben. Man wäscht mit reichlich Wasser und eluiert das Produkt mit einer 10 %igen aqu. Ammoniak-Lösung. Die produkthaltigen Fraktionen werden in Vakuum weitgehend zur Trockne eingedampft.

c) Der Rückstand wird in 200 l Wasser gelöst und 17,62 kg (48,6 mol) Gadoliniumoxid zugegeben. Man kocht 3 Stunden unter Rückfluß. Anschließend setzt man 2 l Eisessig zu und kocht weitere 2 Stunden unter Rückfluß. Es wird 5 kg Aktivkohle zugegeben und 1 h bei 90°C gerührt. Die lösung wird filtriert und das Filtrat mehrmals über eine Ionenaustauscher-Säulen-Kaskade (bestehend aus IRA 67 ($OH^-$-Form)) AMB 252c ($H^+$-Form) gegeben (unter HPLC-Kontrolle). Das Eluat wird auf ein Volumen von 300 l eingeengt und mit je 2 l saurem Ionenaustauscher IR 120 ($H^+$) sowie basischem Austauscher IRA 67 ($OH^-$) 3 Stunden lang gerührt. Man filtriert vom Austauscher ab und wäscht 2 mal mit 10 l Wasser nach. Zum Filtrat setzt man 5 kg Aktivkohle zu und rührt 2 Stunden bei 80°C. Die Lösung wird filtriert und das Filtrat im Vakuum eingeengt. Der Rückstand wird aus Ethanol (ca. 300 l) umkristallisiert. Man saugt den Niederschlag ab, wäscht 1 mal mit 50 l reinem Ethanol nach und trocknet ihn 48 Stunden bei 70°C im Trockenschrank.
Ausbeute: 45,22 kg (67,2 % d. Th.) (auf Wasser korrigiert/bezogen auf Cyclen) farbloses kristallines Pulver

Wassergehalt: 3,5 %

| Elementaranalyse (bezogen auf wasserfreie Substanz): | | | | |
|---|---|---|---|---|
| ber. | C 36,55 | H 5,23 | N 10,03 | Gd 28,15 |
| gef. | C 36,68 | H 5,31 | N 9,91 | Gd 28,03 |

**Beispiel 4**

Dysprosium-Komplex von 10-(2-Hydroxypropyl)-1,4,7-tris(carboxymethyl)-1,4,7,10-tetraazacyclododecan

Analog zu **Beispiel 3c**) kann der entsprechende Dysprosium-Komplex hergestellt werden, wenn anstelle von Gadoliniumoxid entsprechend 18,13 kg (48,6 mol) Dysprosiumoxid umgesetzt werden.
Ausbeute: 47,14 kg (65,3 % d. Th.) (auf Wasser korrigiert/bezogen auf Cyclen)

Wassergehalt: 4,1 %

| Elementaranalyse (bezogen auf wasserfreie Substanz): | | | | |
|---|---|---|---|---|
| ber. | C 36,20 | H 5,18 | N 9,94 | Dy 28,82 |
| gef. | C 36,32 | H 5,27 | N 9,87 | Dy 28,69 |

**Beispiel 5**

Gadolinium-Komplex von 10-(2-Hydroxy-3-methoxy-propyl)-1,4,7-tris(carboxymethyl)-1,4,7,10-tetraazacyclododecan

In analoger Weise kann mit 2,3-Epoxypropylmethylether anstelle von Propylenoxid wie in Beispiel 3 beschrieben, umgesetzt werden. So werden zum Beispiel aus 100 g (0,58 mol) 1,4,7,10-Tetraazacyclododecan 216,7 g (61 % d. Th.) der Titelverbindung als farbloses Pulver erhalten (Kristallisation aus wässrigem Aceton).

Wassergehalt: 3,8 %

| Elementaranalyse (berechnet auf wasserfreie Substanz): | | | | |
|---|---|---|---|---|
| ber.: | C 36,72 | H 5,72 | N 9,52 | Gd 26,71 |
| gef.: | C 36,51 | H 5,83 | N 9,39 | Gd 26,57 |

**Beispiel 6**

Gadolinium-Komplex von 10-(2-Hydroxy-3-benzyloxy-propyl)-1,4,7-tris(carboxymethyl)-1,4,7,10-tetraazacyclododecan

In analoger Weise kann mit 2,3-Epoxypropyl-benzylether anstelle von Propylenoxid wie in Beispiel 3 beschrieben, umgesetzt werden. Bezogen auf 1,4,7,10-Tetraazacyclododecan werden 1,1 Äquivalente vom Epoxid eingesetzt. Man erwärmt 16 Stunden bei 110°C (anstatt 24 Stunden wie in Beispiel 3 beschrieben). So werden aus 100 g (0,58 mol) 1,4,7,10 Tetraazacyclododecan 249,2 g (62 % d. Th.) der Titelverbindung als farbloses Pulver erhalten (Kristallisation aus Isopropanol).

Wassergehalt: 4,2 %

| Elementaranalyse (berechnet auf wasserfreie Substanz): | | | | |
|---|---|---|---|---|
| ber.: | C 43,36 | H 5,31 | N 8,43 | Gd 23,65 |
| gef.: | C 43,21 | H 5,40 | N 8,32 | Gd 23,48 |

**Beispiel 7**

Gadolinium-Komplex von 10-(2,3,4-Trihydroxybutyl)-1,4,7-tris(carboxymethyl)-1,4,7,10-tetraazacyclododecan

In analoger Weise kann mit 2-(2,2-Dimethyl-1,3-dioxolan-4-yl)-ethylenoxid anstelle von 4,4-Dimethyl-3,5,8-trioxabicyclo-(5.1.0)-octan wie in Beispiel 1 beschrieben, umgesetzt werden. Bezogen auf 1,4,7,10-Tetraazacyclododecan werden 1,1 Äquivalente vom Epoxid eingesetzt. Man erwärmt 16 Stunden bei 110°C (anstatt 24 Stunden wie in Beispiel 1 beschrieben). So werden aus 100 g (0,58 mol) 1,4,7,10-Tetraazacyclododecan 232,8 g (64 % d. Th.) der Titelverbindung als farbloses, kristallines Pulver erhalten (Kristallisation aus 90 %igem aqu. Ethanol).

Wassergehalt: 3,5 %

| Elementaranalyse (berechnet auf wasserfreie Substanz): | | | | |
|---|---|---|---|---|
| ber.: | C 35,75 | H 5,17 | N 9,26 | Gd 26,00 |
| gef.: | C 35,55 | H 5,23 | N 9,14 | Gd 25,87 |

**Beispiel 8**

Gadolinium-Komplex von 10-(2-Hydroxy-3-tert.-butoxy-propyl)-1,4,7-tris(carboxymethyl)-1,4,7,10-tetraazacyclododecan

In analoger Weise kann mit 2,3-Epoxypropyl-tert.-butylester anstelle von Propylenoxid wie in Beispiel 3 beschrieben, umgesetzt werden. Bezogen auf 1,4,7,10-Tetraazacyclododecan werden 1,1 Äquivalente vom Epoxid eingesetzt. Man erwärmt 16 Stunden bei 110°C (anstatt 24 Stunden wie in Beispiel 3 beschrieben). So werden aus 100 g (0,58 mol) 1,4,7,10-Tetraazacyclododecan 225 g (59 % d. Th.) der Titelverbindung als farbloses, kristallines Pulver erhalten (Kristallisation aus Aceton/Ethanol)

Wassergehalt: 4,0 %

| Elementaranalyse (berechnet auf wasserfreie Substanz): | | | | |
|---|---|---|---|---|
| ber.: | C 39,99 | H 5,91 | N 8,88 | Gd 24,93 |
| gef.: | C 39,81 | H 6,05 | N 8,73 | Gd 24,82 |

**Beispiel 9**

Gadolinium-Komplex von 10-(2,6,7-Trihydroxy-4-oxaheptyl)-1,4,7-tris(carboxymethyl)-1,4,7,10-tetraazacyclododecan

In analoger Weise kann mit 2,2-Dimethyl-4-(2',3'-epoxy)-propoxy-methyl-1,3-dioxolan anstelle von 4,4-Dimethyl-3,5,8-trioxabicyclo-(5.1.0)-octan wie in Beispiel 1 beschrieben, umgesetzt werden. Bezogen auf 1,4,7,10-Tetraazacyclododecan werden 1,1 Äquivalente vom Epoxid eingesetzt. Man erwärmt 16 Stunden bei 110°C (anstatt 24 Stunden wie in Beispiel 1 beschrieben). So werden aus 100 g (0,58 mol) 1,4,7,10-Tetraazacyclododecan 242,2 g (62 % d. Th.) der Titelverbindung als farbloses, kristallines Pulver erhalten (Kristallisation aus Ethanol).

Wassergehalt: 3,6 %

| Elementaranalyse (berechnet auf wasserfreie Substanz): | | | | |
|---|---|---|---|---|
| ber.: | C 37,03 | H 5,44 | N 8,64 | Gd 24,24 |
| gef.: | C 36,91 | H 5,58 | N 8,49 | Gd 24,13 |

**Beispiel 10**

Gadolinium-Komplex von 10-(2-Hydroxy-3-isopropoxy-propyl)-1,4,7-tris(carboxymethyl)-1,4,7,10-tetraazacyclododecan

In analoger Weise kann mit 2,3-Epoxypropylisopropylether anstelle von Propylenoxid wie in Beispiel 3 beschrieben, umgesetzt werden. Bezogen auf 1,4,7,10-Tetraazacyclododecan werden 1,1 Äquivalente vom Epoxid eingesetzt. Man erwärmt 16 Stunden bei 110°C (anstatt 24 Stunden wie in Beispiel 3 beschrieben). So werden aus 100 g (0,58 mol) 1,4,7,10-Tetraazacyclododecan 232,5 g (63 % d. Th.) der Titelverbindung als farbloses, kristallines Pulver erhalten (Kristallisation aus Isopropanol).

Wassergehalt: 3,1 %

| Elementaranalyse (berechnet auf wasserfreie Substanz): | | | | |
|---|---|---|---|---|
| ber.: | C 38,95 | H 5,72 | N 9,08 | Gd 25,50 |
| gef.: | C 38,85 | H 5,81 | N 8,93 | Gd 25,35 |

**Beispiel 11**

Gadolinium-Komplex von 10-(2-Hydroxy-2-methyl-propyl)-1,4,7-tris(carboxymethyl)-1,4,7,10-tetraazacyclododecan

In analoger Weise kann mit iso-Butylenoxid anstelle von Propylenoxid wie in Beispiel 3 beschrieben, umgesetzt werden. So werden zum Beispiel aus 100 g (0,58 mol) 1,4,7,10-Tetraazacyclododecan 209,4 g (60 % d. Th.) der Titelverbindung als farbloses Pulver erhalten (Kristallisation aus wässrigem Aceton).

Wassergehalt: 4,0 %

| Elementaranalyse (berechnet auf wasserfreie Substanz): | | | | |
|---|---|---|---|---|
| ber.: | C 37,75 | H 5,46 | N 9,78 | Gd 27,46 |
| gef.: | C 37,61 | H 5,53 | N 9,70 | Gd 27,38 |

**Beispiel 12**

Gadolinium-Komplex von 10-(2-Hydroxy-3-phenoxy-propyl)-1,4,7-tris(carboxymethyl)-1,4,7,10-tetraazacyclododecan

In analoger Weise kann mit 2,3-Epoxypropyl-phenylether anstelle von Propylenoxid wie in Beispiel 3 beschrieben, umgesetzt werden. Bezogen auf 1,4,7,10-Tetraazacyclododecan werden 1,1 Äquivalente vom Epoxid eingesetzt. Man erwärmt 16 Stunden bei 110°C (anstatt 24 Stunden wie in Beispiel 3 beschrieben). So werden aus 100 g (0,58 mol) 1,4,7,10-Tetraazacyclododecan 252,4 g (64 % d. Th.) der Titelverbindung als farbloses, kristallines Pulver erhalten (Kristallisation aus wässrigem Aceton).

Wassergehalt: 3,7 %

| Elementaranalyse (berechnet auf wasserfreie Substanz): | | | | |
|---|---|---|---|---|
| ber.: | C 43,49 | H 5,02 | N 8,45 | Gd 23,73 |
| gef.: | C 43,31 | H 5,11 | N 8,38 | Gd 23,65 |

**Patentansprüche**

1. Verfahren zur Herstellung von Metall-Komplexen der N-ß-Hydroxyalkyl-tri-N-carboxyalkyl-1,4,7,10-tettaazacyclododecan- und N-ß-Hydroxyalkyl-tri-N-carboxyalkyl-1,4,8,11-tetraazacyclotetradecan-Derivate der allgemeinen Formel I

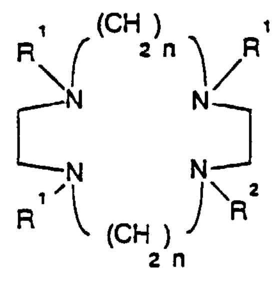

(I)

worin

$R^1$ $-CH_2-COOY$
Y Wasserstoff, ein Metallionenäquivalent eines Elementes des Ordnungszahlen 21-32, 37-39, 42-51 oder 57-83

mit der Maßgabe, daß mindestens zwei Substituenten Y für Metalläquivalente stehen,

n die Ziffern 2 oder 3

$R^2$ eine

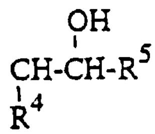

-Gruppe wobei

$R^4$ und $R^5$ unabhängig voneinander jeweils für ein Wasserstoffatom, einen gegebenenfalls durch 1 bis 10 Sauerstoffatom(e), eine Phenylen-, Phenylenoxy- oder Phenylendioxygruppe unterbrochenen $C_1$-$C_{20}$-Alkylrest, der gegebenenfalls durch 1 bis 3 $C_1$-$C_6$-Alkyl-, 1-3 Trifluormethyl-, 1 bis 7 Hydroxy-, 1 bis 3 $C_1$-$C_7$-Alkoxy- oder ($C_6$-$C_{10}$)-Ar($C_1$-$C_4$)alkoxy-, 1 bis 2 $CO_2R^6$-Reste, wobei

$R^6$ für ein Wasserstoffatom, eine $C_1$-$C_6$-Alkylgruppe, eine $C_6$-$C_{10}$-Aryl- oder eine $C_6$-$C_{10}$-Ar($C_1$-$C_4$)alkylgruppe steht,

stehen,

bedeuten, wobei die gegebenenfalls vorhandenen Hydroxyreste gegebenenfalls in geschützter Form vorliegen sowie deren Salze mit anorganischen und/oder organischen Basen, Aminosäuren oder Aminosäureamiden, enthaltend die Umsetzung von Intermediaten der allgemeinen Formel V

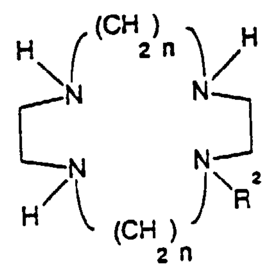

(V),

worin $R^2$ und n die oben genannten Bedeutungen haben,

zu Verbindungen der allgemeinen Formel VII

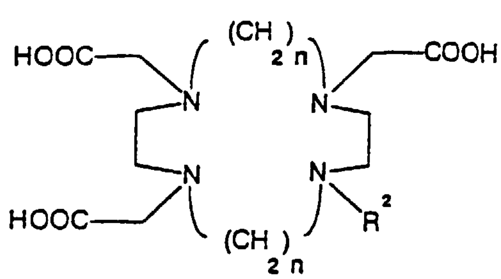

(VII),

worin $R^2$ und n die oben genannten Bedeutungen haben,

dadurch gekennzeichnet, daß die aus 1,4,7,10-Tetraazacyclododecan oder 1,4,8,11-Tetraazacyclotetradecan erhaltene Verbindung der allgemeinen Formel II

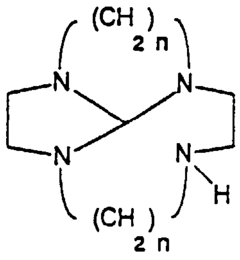

(II)

worin

n für die Ziffern 2 oder 3 steht
mit einem Epoxid der allgemeinen Formel III

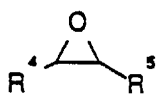

(III),

worin
$R^4$ und $R^5$ die oben angegebene Bedeutung haben, wobei gegebenenfalls vorhandene Hydroxy- oder Carboxygruppen gegebenenfalls geschützt sind, zu einem Intermediat der allgemeinen Formel IV

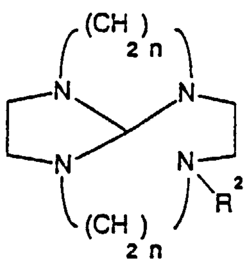

(IV),

worin
$R^2$ die oben angegebene Bedeutung hat,
wobei gegebenenfalls vorhandene Hydroxgruppen und/oder Carboxygruppen gegebenenfalls geschützt sind, umgesetzt wird,
dieses zu einem Intermediat V verseift wird

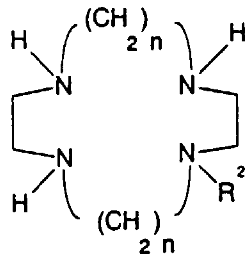

(V),

und, dieses in Gegenwart eines Säurefängers, mit einer Verbindung der Formel VI

$$X\text{-}CH_2\text{-}COOZ \qquad (VI),$$

worin

X eine Abgangsgruppe,
Z Wasserstoff, eine Carboxyschutzgruppe oder ein Metallkation

bedeuten,
gegebenenfalls nach Schutz von in (V) gegebenenfalls vorhandenen Hydroxy- oder Carboxygruppen in einem polaren Lösungsmittel bei -10°C bis 170°C innerhalb von 1-100 Stunden umgesetzt wird, gegebenenfalls Schutzgruppen abgespalten werden und der so erhaltene Komplexbildner der Formel VII,

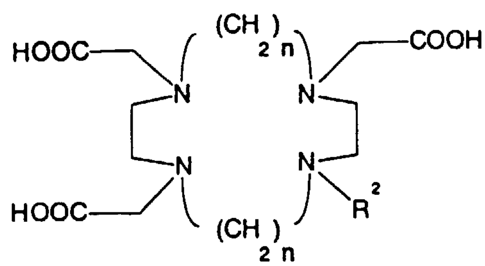

(VII)

mit einem Metalloxid oder Metallsalz eines Elementes der Ordnungszahlen 21-32, 37-39, 42-51 oder 57-83 umgesetzt wird und gegebenenfalls noch vorhandene Wasserstoffatome durch Kationen anorganischer und/oder organischer Basen, Aminosäuren oder Aminosäureamide substituiert werden oder die noch vorhandenen Säuregruppen ganz oder teilweise in Ester oder Amide überführt werden.

2. Verfahren gemäß Anspruch 1, dadurch gekennzeichnet, daß die Umsetzung zu einer Verbindung der allgemeinen Formel (IV) bei einer Temperatur zwischen 20°C und 210°C ohne Lösungsmittel oder in einem aprotischen Lösungsmittel erfolgt.

3. Verfahren gemäß Anspruch 1, dadurch gekennzeichnet, daß die Umsetzung zu einer Verbindung der allgemeinen Formel (V) bei Temperaturen zwischen 20°C und 120°C in wässrigen Alkoholen unter Zusatz von Natriumhydroxid oder Kaliumhydroxid oder Salzsäure erfolgt.

4. Verfahren gemäß Anspruch 1, dadurch gekennzeichnet, daß die Verbindung der Formel (VI) Chloressigsäure ist.

5. Verfahren gemäß Anspruch 1, dadurch gekennzeichnet, daß die Umsetzung zu einer Verbindung der allgemeinen Formel (VII) ohne Schutz von Hydroxy- oder Carboxygruppen bei Temperaturen zwischen 40°C und 100°C in Wasser innerhalb von 3-24 Stunden erfolgt.

6. Verfahren gemäß Anspruch 1, dadurch gekennzeichnet, daß eine Verbindung der Formel (I)

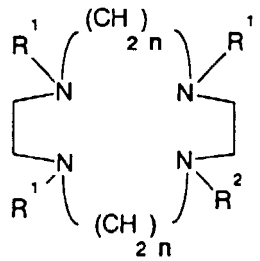

(I)

worin

$R^1$ $-CH_2-COOY$
Y ein Metallionenäquivalent eines Elementes des Ordnungszahlen 21-32, 37-39, 42-51 oder 57-83
n die Ziffer 2
$R^2$ eine

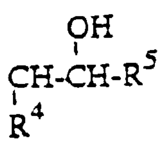

-Gruppe

$R^4$ und $R^5$ unabhängig voneinander jeweils für ein Wasserstoffatom, einen gegebenenfalls durch 1 bis 5 Sauerstoffatom(e), eine Phenylen-, Phenylenoxy- oder Phenylendioxygruppe unterbrochenen $C_1$-$C_{10}$-Alkylrest, der gegebenenfalls durch 1 bis 3 $C_1$-$C_6$-Alkyl-, 1-3-Trifluormethyl-, 1 bis 5 Hydroxy-, 1 bis 3 $C_1$-$C_7$-Alkoxy- oder 1 bis $CO_2R^6$-Reste, wobei

$R^6$ für ein Wasserstoffatom, eine $C_1$-$C_6$-Alkylgruppe, oder eine Benzylgruppe steht,

stehen,
bedeuten, erhalten wird.

**7.** Verfahren gemäß Anspruch 1 zur Herstellung von Metallkomplexen von 10-(1-Hydroxymethyl-2,3-dihydroxypropyl)-1,4,7-tris(carboxymethyl)-1,4,7,10-tetraazacyclododecan.

**8.** Verfahren gemäß Anspruch 1 zur Herstellung von Metallkomplexen von 10-(2-Hydroxypropyl)-1,4,7-tris(carboxymethyl)-1,4,7,10-tetraazacyclododecan.

**9.** Verfahren gemäß Anspruch 1 zur Herstellung von Gadolinium- oder Dysprosium-Komplexen von 10-(1-Hydroxymethyl-2,3-dihydroxypropyl)-1,4,7-tris(carboxymethyl)-1,4,7,10-tetraazacyclododecan.

**10.** Verfahren gemäß Anspruch 1 zur Herstellung von Gadolinium- oder Dysprosium-Komplexen von 10-(2-Hydroxypropyl)-1,4,7-tris(carboxymethyl)-1,4,7,10-tetraazacyclododecan.

**11.** Verfahren zur Herstellung von Metall-Komplexen der N-(2-Hydroxy-2-methyl-propyl)-tri-N-carboxyalkyl-1,4,7,10-tetraazacyclododecan- oder N-(2-Hydroxy-2-methyl-propyl)-tri-N-carboxyalkyl-1,4,8,11-tetraazacyclotetradecan-Derivaten der allgemeinen Formel Ia

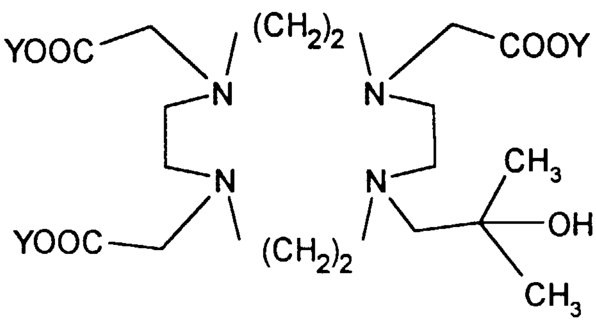

(Ia)

worin

Y Wasserstoff oder ein Metallionenäquivalent des Gadoliniums mit der Maßgabe, daß mindestens zwei Substituenten Y für Metalläquivalente des Gadoliniums stehen,

bedeuten, sowie deren Salze mit anorganischen und/oder organischen Basen, Aminosäuren oder Aminosäureamiden,
dadurch gekennzeichnet, daß die aus 1,4,7,10-Tetraazacyclododecan oder 1,4,8,11-Tetraazacyclotetradecan erhaltene Verbindung der allgemeinen Formel IIa

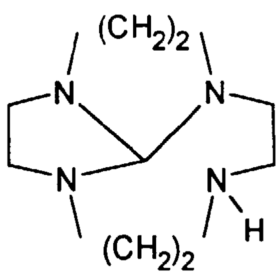

(IIa)

mit Isobutylenoxid zu einem Intermediat der allgemeinen Formel IVa

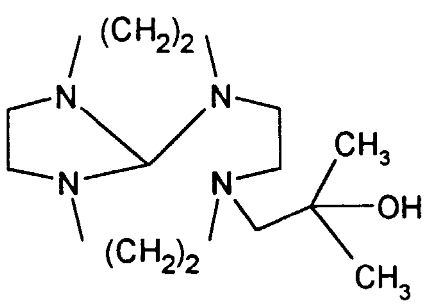

(IVa)

umgesetzt wird,
dieses zu einem Intermediat Va verseift wird

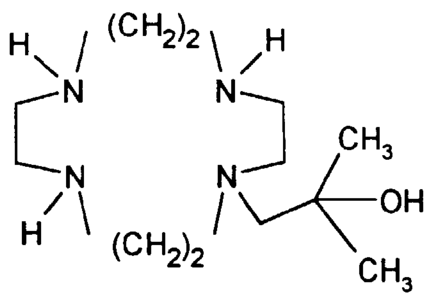

(Va),

und dieses in Gegenwart eines Säurefängers, mit einer Verbindung der Formel VI

$$X\text{-}CH_2COOZ \qquad (VI),$$

worin

X eine Abgangsgruppe,

Z Wasserstoff, eine Carboxyschutzgruppe oder ein Metallkation

bedeuten,
in einem polaren Lösungsmittel bei -10°C bis 170°C innerhalb von 1 bis 100 Stunden umgesetzt wird und der so erhaltene Komplexbildner der Formel VIIa

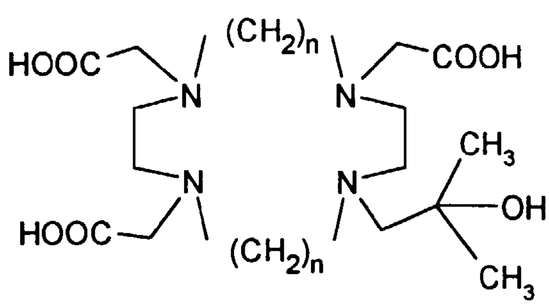

(VIIa)

mit Gadoliniumoxid oder einem Gadoliniumsalz umgesetzt wird und gegebenfalls noch vorhandene Wasserstoffatome durch Kationen anorganischer Basen, Aminosäuren oder Aminosäureamide substituiert werden oder die noch vorhandenen Säuregruppen ganz oder teilweise in Ester oder Amide überführt werden.

**Claims**

1. A process for the manufacture of metal complexes of N-ß-hydroxyalkyl-tri-N-carboxyalkyl-1,4,7,10-tetraazacyclododecane derivatives and N-β-hydroxyalkyl-tri-N-carboxyalkyl-1,4,8,11-tetraazacyclotetradecane derivatives of the general formula I

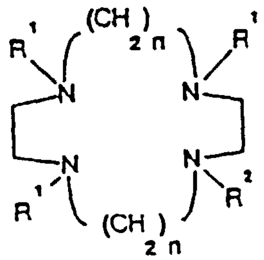

(I),

wherein

$R^1$ represents $-CH_2-COOY$,
Y represents hydrogen or an equivalent of a metal ion of an element of atomic number 21-32, 37-39, 42-51 or 57-83, with the proviso that at least two substituents Y represent metal equivalents,
n is the number 2 or 3,
$R^2$ represents a

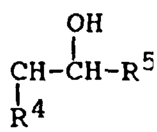

group,

$R^4$ and $R^5$ each independently of the other representing a hydrogen atom or a $C_1$-$C_{20}$-alkyl radical that is optionally interrupted by from 1 to 10 oxygen atom(s) or by a phenylene, phenyleneoxy or phenylenedioxy group and that is optionally substitued by from 1 to 3 $C_1$-$C_6$-alkyl, from 1 to 3 trifluoromethyl, from 1 to 7 hydroxy, from 1 to 3 $C_1$-$C_7$-alkoxy or ($C_6$-$C_{10}$)-Ar($C_1$-$C_4$)-alkoxy or 1 or 2 $CO_2R^6$ radicals,

$R^6$ representing a hydrogen atom, a $C_1$-$C_6$-alkyl group, a $C_6$-$C_{10}$-aryl group or a $C_6$-$C_{10}$-Ar ($C_1$-$C_4$)alkyl group,

the optionally present hydroxy radicals being optionally in protected form,
and salts thereof with inorganic and/or organic bases, amino acids or amino acid amides,
comprising the reaction of intermediates of the general formula V

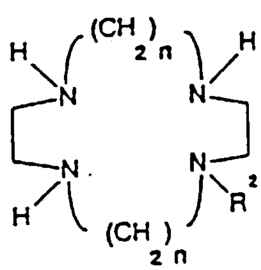

(V),

wherein $R^2$ and n have the meanings mentioned above,
to form compounds of the general formula VII

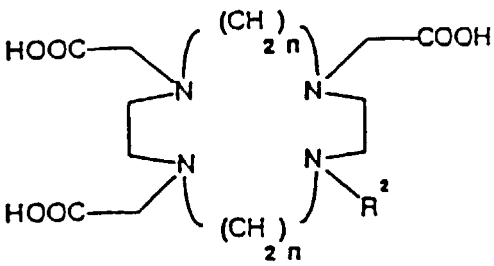

(VII),

wherein $R^2$ and n have the meanings mentioned above,

characterised in that the compound of the general formula II

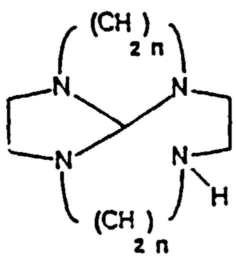

(II),

wherein

n is the number 2 or 3,
which is obtained from 1,4,7,10-tetraazacyclododecane or 1,4,8,11-tetraazacyclotetradecane, is reacted with an epoxide of the general formula III

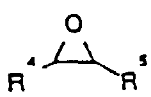

(III),

wherein
$R^4$ and $R^5$ have the meanings given above, optionally present hydroxy or carboxy groups being optionally protected, to form an intermediate of the general formula IV

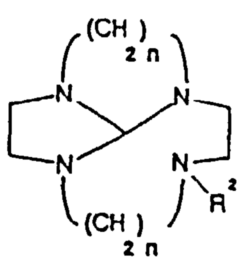

(IV),

wherein
$R^2$ has the meaning given above,
optionally present hydroxy groups and/or carboxy groups being optionally protected,

that intermediate is hydrolysed to form an intermediate V

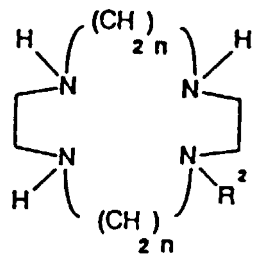

(V),

and that intermediate is reacted, in the presence of an acid acceptor, with a compound of the formula VI

$$X\text{-}CH_2\text{-}COOZ \qquad (VI),$$

wherein

X represents a leaving group,
Z represents hydrogen, a carboxy-protecting group or a metal cation,

optionally after protection of hydroxy or carboxy groups optionally present in (V), in a polar solvent at from -10°C to 170°C within a period of from 1 to 100 hours, where applicable protecting groups are removed, and the complex former of formula VII so obtained,

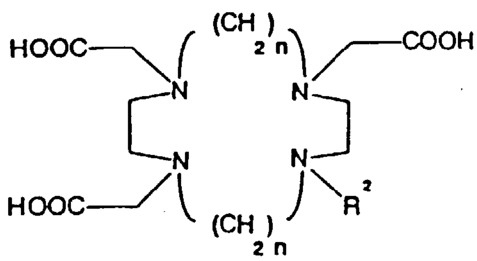

(VII),

is reacted with a metal oxide or metal salt of an element of atomic number 21-32, 37-39, 42-51 or 57-83 and optionally hydrogen atoms still present are replaced by cations of inorganic and/or organic bases, amino acids or amino acid amides, or the acid groups still present are completely or partly converted into esters or amides.

**2.** A process according to claim 1, characterised in that the reaction to form a compound of the general formula (IV) takes place at a temperature of from 20°C to 210°C without solvent or in an aprotic solvent.

**3.** A process according to-the claim 1, characterised in that the reaction to form a compound of the general formula (V) takes place at temperatures of from 20°C to 120°C in aqueous alcohols with the addition of sodium hydroxide or potassium hydroxide or hydrochloric acid.

**4.** A process according to claim 1, characterised in that the compound of the formula (VI) is chloroacetic acid.

**5.** A process according to claim 1, characterised in that the reaction to form a compound of the general formula (VII) takes place without protection of hydroxy or carboxy groups, at temperatures of from 40°C to 100°C, in water, within a period of from 3 to 24 hours.

**6.** A process according to claim 1, characterised in that a compound of the formula (I)

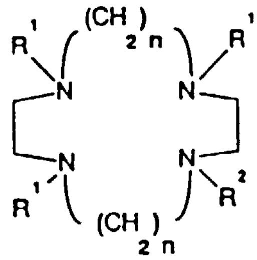

(I),

wherein

$R^1$ represents $-CH_2-COOY$,
Y represents an equivalent of a metal ion of an element of atomic number 21-32, 37-39, 42-51 or 57-83,
n is the number 2,
$R^2$ represents a

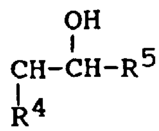

group,

$R^4$ and $R^5$ each independently of the other represents a hydrogen atom, a $C_1$-$C_{10}$-alkyl radical that is optionally interrupted by from 1 to 5 oxygen atom(s) or by a phenylene, phenyleneoxy or phenylenedioxy group and that is optionally substituted by from 1 to 3 $C_1$-$C_6$-alkyl, from 1 to 3 trifluoromethyl, from 1 to 5 hydroxy, from 1 to 3 $C_1$-$C_7$-alkoxy or 1 or 2 $CO_2R^6$ radicals,

$R^6$ representing a hydrogen atom, a $C_1$-$C_6$-alkyl group or a benzyl group,

is obtained.

**7.** A process according to claim 1 for the manufacture of metal complexes of 10-(1-hydroxymethyl-2,3-dihydroxypropyl)-1,4,7-tris(carboxymethyl)-1,4,7,10-tetraazacyclododecane.

**8.** A process according to claim 1 for the manufacture of metal complexes of 10-(2-hydroxypropyl)-1,4,7-tris-(carboxymethyl)-1,4,7,10-tetraazacyclododecane.

**9.** A process according to claim 1 for the manufacture of gadolinium or dysprosium complexes of 10-(1-hydroxymethyl-2,3-dihydroxypropyl)-1,4,7-tris(carboxymethyl)-1,4,7,10-tetraazacyclododecane.

**10.** A process according to claim 1 for the manufacture of gadolinium or dysprosium complexes of 10-(2-hydroxypropyl)-1,4,7-tris(carboxymethyl)-1,4,7,10-tetraazacyclododecane.

**11.** A process for the manufacture of metal complexes of N-(2-hydroxy-2-methyl-propyl)-tri-N-carboxyalkyl-1,4,7,10-tetraazacyclododecane derivatives or N-(2-hydroxy-2-methyl-propyl)-tri-N-carboxyalkyl-1,4,8,11-tetraazacyclomethyl-propyl)-tri-N-carboxyalkyl-1,4,8,11-tetraazacyclotetradecane derivatives of the general formula Ia

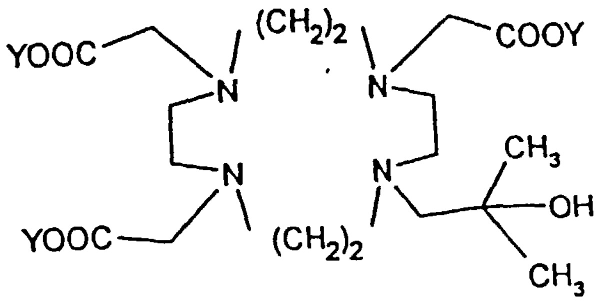

(Ia),

wherein

Y represents hydrogen or a metal ion equivalent of gadolinium, with the proviso that at least two substituents Y represent metal equivalents of gadolinium,

and salts thereof with inorganic and/or organic bases, amino acids or amino acid amides, characterised in that the compound of the general formula IIa

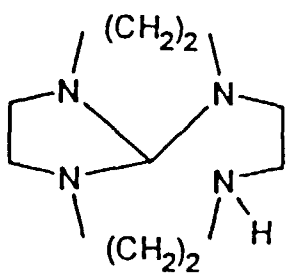

(IIa),

which is obtained from 1,4,7,10-tetraazacyclododecane or 1,4,8,11-tetraazacyclotetradecane, is reacted with isobutylene oxide to form an intermediate of the general formula IVa

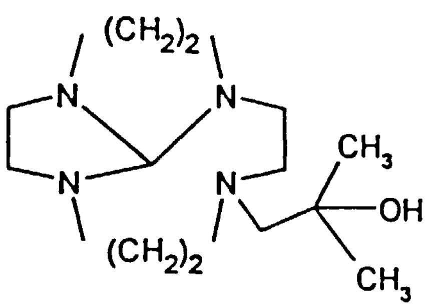

(IVa),

that intermediate is hydrolysed to form an intermediate Va

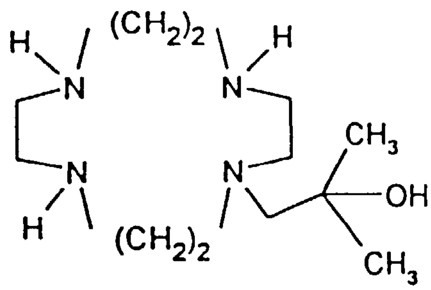

(Va),

and that intermediate is reacted, in the presence of an acid acceptor, with a compound of the formula VI

$$X\text{-}CH_2COOZ \qquad (VI),$$

wherein

X represents a leaving group, and
Z represents hydrogen, a carboxy-protecting group or a metal cation,

in a polar solvent at from -10°C to 170°C within a period of from 1 to 100 hours, and the complex former of the formula VIIa so obtained,

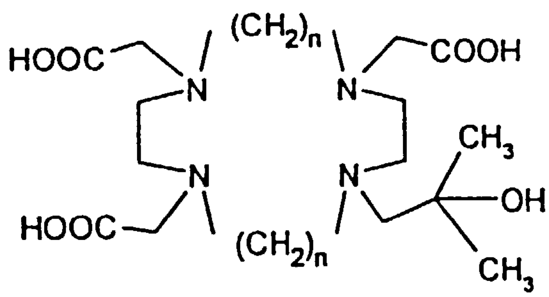

(VIIa),

is reacted with gadolinium oxide or a gadolinium salt and optionally hydrogen atoms still present are replaced by cations of inorganic bases, amino acids or amino acid amides or the acid groups still present are completely or partly converted into esters or amides.

## Revendications

1. Procédé pour la préparation de complexes métalliques des dérivés du N-ß-hydroxyalkyle-tri-N-carboxyalkyl-1,4,7,10-tétraazacyclododécane et du N-ß-hydroxyalkyle-tri-N-carboxyalkyl-1,4,8,11-tétraazacyclododécane de formule générale I

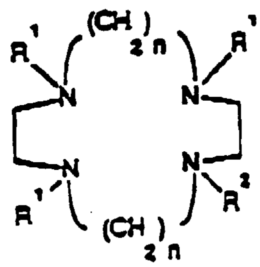

(I)

dans laquelle

$R^1$ représente $-CH_2-COOY$

Y représente l'hydrogène, un équivalent d'ion métallique d'un élément ayant un nombre atomique 21 à 32, 37 à 39, 42 à 51 ou 57 à 83, à la condition qu'au moins deux substituants Y représentent des équivalents de métaux,

n vaut 2 ou 3

$R^2$ représente un groupe

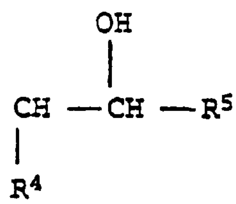

où

$R^4$ et $R^5$, indépendamment l'un de l'autre, représentent chacun un atome d'hydrogène, un radical alkyle en $C_1$-$C_{20}$, éventuellement interrompu par 1 à 10 atome(s) d'oxygène, un groupe phénylène, phénylènoxy ou phénylènedioxy, lequel radical alkyle pouvant être éventuellement substitué par 1 à 3 radicaux alkyle en $C_1$-$C_6$, 1 à 3 radicaux trifluorométhyle, 1 à 7 radicaux hydroxyle, 1 à 3 radicaux alcoxy en $C_1$-$C_7$, ou (Ar en $C_6$-$C_{10}$)-alcoxy en $C_1$-$C_4$, 1 à 2 radicaux $CO_2R^6$,

où

$R^6$ représente un atome d'hydrogène, un groupe alkyle en $C_1$-$C_6$, un groupe aryle en $C_6$-$C_{10}$ ou un groupe (Ar en $C_6$-$C_{10}$)-alkyle en $C_1$-$C_4$,

les radicaux hydroxyles éventuellement présents pouvant éventuellement être sous forme protégée, ainsi que leurs sels avec des amides d'amino-acides, amino-acides ou bases inorganiques et/ou organiques,
comprenant la réaction des intermédiaires de formule générale V

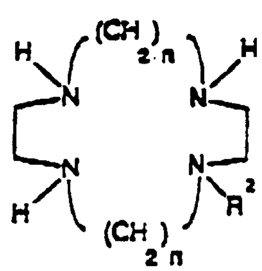

(V),

dans laquelle $R^2$ et n ont les significations données ci-dessus,

pour obtenir des composés de formule générale VII

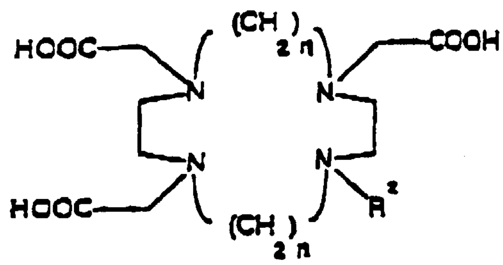

(VII),

dans laquelle $R^2$ et n ont les significations données ci-dessus,

caractérisé en ce qu'on fait réagir le composé obtenu à partir du 1,4,7,10-tétraazacyclododécane ou du 1,4,8,11-tétraazacyclotétradécane, répondant à la formule générale II

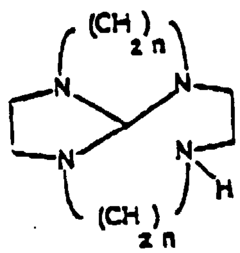

(II)

dans laquelle

n vaut 2 ou 3,

avec un époxyde de formule générale III

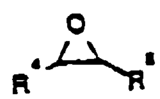

(III),

dans laquelle

$R^4$ et $R^5$ ont la signification donnée ci-dessus, les groupes hydroxyle ou carboxyle éventuellement présents étant éventuellement protégés, pour obtenir un intermédiaire de formule générale IV

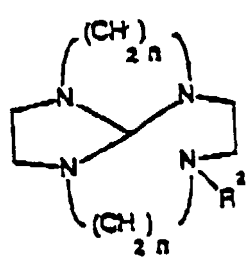

(IV),

dans laquelle
$R^2$ a la signification donnée ci-dessus,
les groupes hydroxyles et/ou carboxyles éventuellement présents étant éventuellement protégés,
en saponifiant celui-ci en un intermédiaire de formule V

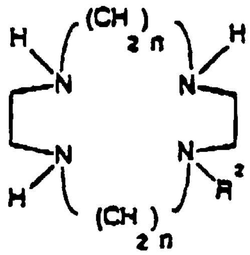

(V).

et, en faisant réagir celui-ci, en présence d'un piège à acides, avec un composé de formule VI

$$X\text{-}CH_2\text{-}COOZ \qquad (VI)$$

dans laquelle

X représente un groupe éliminable,
Z représente l'hydrogène, un groupe protecteur de carboxyle ou un ion métallique,

éventuellement après protection des groupes hydroxyles ou carboxyles éventuellement présents dans (V), dans un solvant polaire à une température de -10°C à 170°C, en l'espace de 1 à 100 heures, en éliminant éventuellement les groupes protecteurs et en faisant réagir l'agent complexant de formule VII

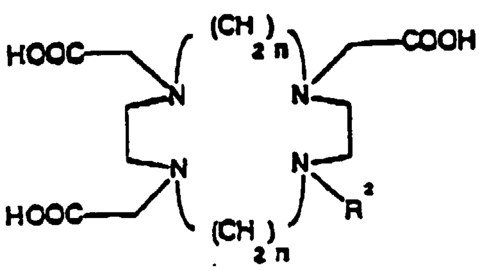

(VII)

avec un oxyde métallique ou un sel métallique d'un élément ayant un nombre atomique de 21 à 32, 37 à 39, 42 à 51 ou 57 à 83 et éventuellement en transformant les atomes d'hydrogène encore présents par des cations d'amides d'amino-acides, des amino-acides ou des bases inorganiques et/ou organiques, ou bien les groupes acides encore présents, entièrement ou partiellement en esters ou amides.

2. Procédé selon la revendication 1, caractérisé en ce qu'on réalise la réaction pour obtenir un composé de formule générale (IV) à une température comprise entre 20°C et 210°C, sans solvant ou dans un solvant aprotique.

3. Procédé selon la revendication 1, caractérisé en ce qu'on réalise la réaction pour obtenir un composé de formule générale (V) à une température comprise entre 20°C et 120°C en milieux d'alcools aqueux, en ajoutant de l'hydroxyde de sodium ou de l'hydroxyde de potassium ou de l'acide chlorhydrique.

4. Procédé selon la revendication 1, caractérisé en ce que le composé de formule (VI) est l'acide chloracétique.

5. Procédé selon la revendication 1, caractérisé en ce qu'on réalise la réaction pour obtenir un composé de formule générale (VII) sans protection des groupes hydroxyles ou carboxyles à une température comprise entre 40°C et 100°C, dans l'eau en l'espace de 3 à 24 heures.

6. Procédé selon la revendication 1, caractérisé en ce qu'on obtient un composé de formule (I)

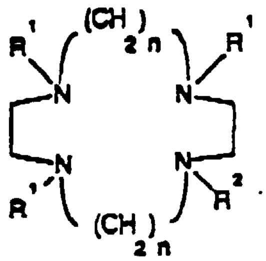

(I)

dans laquelle

$R^1$ représente $-CH_2-COOY$

Y représente un équivalent d'ion métallique d'un élément ayant un nombre atomique 21 à 32, 37 à 39, 42 à 51 ou 57 à 83,

n vaut 2

$R^2$ représente un groupe

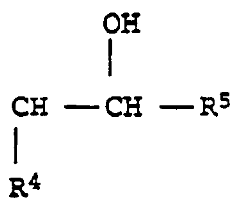

où

$R^4$ et $R^5$, indépendamment l'un de l'autre, représentent chacun un atome d'hydrogène, un radical alkyle en $C_1$-$C_{10}$, éventuellement interrompu par 1 à 5 atome(s) d'oxygène, un groupe phénylène, phénylènoxy ou phénylènedioxy, lequel radical alkyle pouvant être évantuellement substitué par 1 à 3 radicaux alkyle en $C_1$-$C_6$, 1 à 3 radicaux trifluorométhyle, 1 à 5 radicaux hydroxyle, 1 à 3 radicaux alcoxy en $C_1$-$C_7$ ou 1 à 2 radicaux $CO_2R^6$,

où

$R^6$ représente un atome d'hydrogène, un groupe alkyle en $C_1$-$C_6$, un groupe benzyle.

**7.** Procédé selon la revendication 1 pour la préparation de complexes métalliques du 10-(1-hydroxyméthyl-2,3-dihydroxypropyl)-1,4,7-tris(carboxyméthyl)-1,4,7,10-tétra-azacyclododécane.

**8.** Procédé selon la revendication 1 pour la préparation de complexes métalliques du 10-(2-hydroxypropyl)-1,4,7-tris (carboxyméthyl)-1,4,7,10-tétraazacyclododécane.

**9.** Procédé selon la revendication 1 pour la préparation de complexes de gadolinium ou de dysprosium du 10-(1-hydroxyméthyl-2,3-dihydroxypropyl)-1,4,7-tris(carboxyméthyl)-1,4,7,10-tétraazacyclododécane.

**10.** Procédé selon la revendication 1 pour la préparation de complexes de gadolinium ou de dyspropsium du 10-(2-hydroxypropyl)-1,4,7-tris(carboxyméthyl)-1,4,7,10-tétraazacyclododécane.

**11.** Procédé pour la préparation de complexes métalliques des dérivés du N-(2-hydroxy-2-méthyl-propyl)-tri-N-carboxyalkyl-1,4,7,10-tétraazacyclododécane ou du N-(2-hydroxy-2-méthyl-propyl)-tri-N-carboxyalkyl-1,4,8,11-tétraazacyclo-dodécane de formule générale la

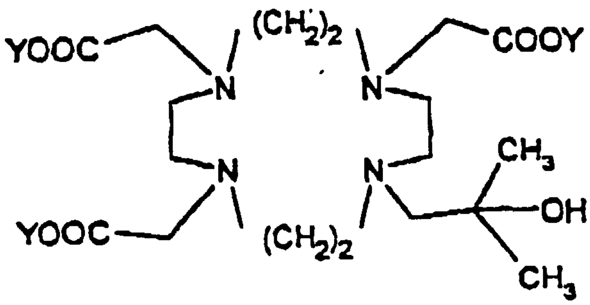

(Ia)

dans laquelle

Y représente l'hydrogène ou un équivalent d'ion gadolinium à la condition au'au moins deux des substituants Y soient des équivalents métal du gadolinium,

ainsi que leurs sels avec des amides d'amino-acides, des amino-acides ou des bases inorganiques et/ou organiques, caractérisé en ce qu'on fait réagir le composé obtenu à partir du 1,4,7,10-tétraazacyclododécane ou du 1,4,8,11-tétraazacyclotétradécane de formule générale IIa

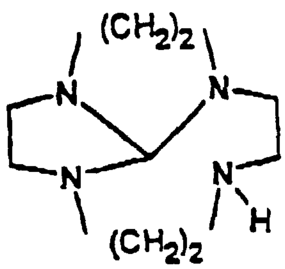

(IIa)

avec l'oxyde d'isobutylène pour obtenir un intermédiaire de formule générale IVa

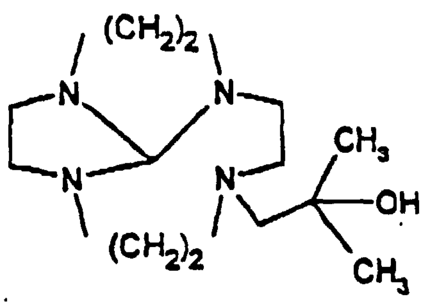

(IVa)

que l'on saponifie pour obtenir un intermédiaire Va

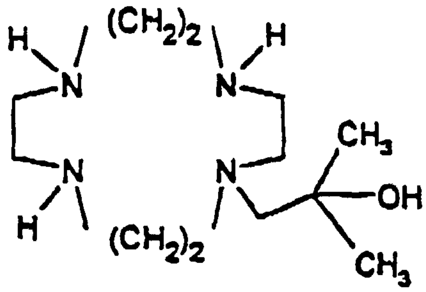

(Va),

et que l'on fait réagir en présence d'un piège à acides, avec un composé de formule VI

$$X\text{-}CH_2\text{-}COOZ \qquad (VI)$$

dans laquelle

X représente un groupe éliminable,
Z représente l'hydrogène, un groupe protecteur de carboxyle ou un ion métallique,

dans un solvant polaire, à une température de -10°C à 170°C, en l'espace de 1 à 100 heures et on fait réagir l'agent complexant ainsi obtenu de formule VIIa

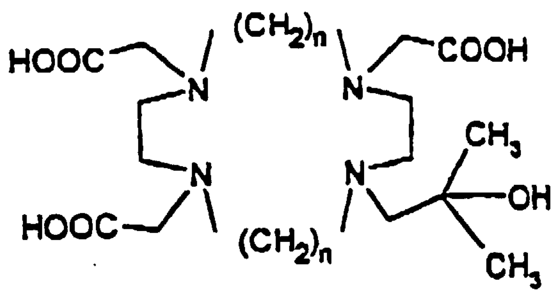

(VIIa)

avec l'oxyde de gadolinium ou un sel de gadolinium et en substituant les atomes d'hydrogène éventuellement encore présents par des cations d'amides d'amino-acides, des amino-acides ou des bases inorganiques ou en transformant en partie ou entièrement les groupes acides éventuellement présents en esters ou amides.